# EUROPEAN PATENT APPLICATION

(11) **EP 4 691 504 A1**
(43) Date of publication of application: **11.02.2026**
(21) Application number: 24780370.3
(22) Date of filing: 26.03.2024
(51) Int. Cl.: A61L 27/02, A61L 27/10

(54) **BIOLOGICAL MATERIAL, POWDER COMPOSITION, BONE PROSTHETIC MATERIAL, AND METHOD FOR USING COMPOSITION CONTAINING COMPOSITE COMPOUND AS BIOLOGICAL MATERIAL**

(30) Priority: 27.03.2023 JP 2023050297
(71) Applicant: Denka Company Limited, Tokyo 103-8338 (JP)
(72) Inventor: MIYATA Kenji, Tokyo 103-8338 (JP); EMOTO Hideyuki, Tokyo 103-8338 (JP)
(74) Representative: Hoffmann Eitle
(86) International application number: PCT/JP2024/012071
(87) International publication number: WO 2024/204262

(57) **Abstract**

A biological material according to the present disclosure includes as a main component, a composite compound having yttrium, oxygen, silicon, and nitrogen as constituent elements. A powder composition according to the present disclosure includes, as a main component, a composite compound having yttrium, oxygen, silicon, and nitrogen as constituent elements, the powder composition having a BET specific surface area of 0.3 m²/g or more. A bone prosthetic material according to the present disclosure includes the biological material described above. A method for using a composition containing a composite compound as a main component as a biological material according to the present disclosure uses, as a biological material, a composition containing, as a main component, a composite compound having yttrium, oxygen, silicon, and nitrogen as constituent elements.

## Description

### TECHNICAL FIELD

The present disclosure relates to: a biological material; a powder composition; a bone prosthetic material; and a method for using a composition containing a composite compound as a biological material.

### BACKGROUND

Silicon nitride is known for having antibacterial properties and biocompatibility. Patent Document 1 proposes changing surface chemical characteristics in order to improve the antibacterial properties of a biomedical implant block which includes a silicon nitride ceramic material. Patent Document 2 proposes: filling silicon nitride in a biomedical implant of polyether ether ketone (PEEK), or the like; and providing a silicon nitride coating. However, when silicon nitride is fired with an oxide, a composite oxide is generated. For example, it is known that when a silicon nitride powder is mixed with an auxiliary agent including yttrium oxide and sintered, a composite oxide such as melilite, YAM, apatite, wollastonite, etc., is generated at a grain boundary of the silicon nitride particle (see, for example, Patent Document 3).

### CITATION LIST

### PATENT LITERATURE

Patent Document 1: JP 2015-516239 A
Patent Document 2: JP 2020-512072 A
Patent Document 3: JP 2000-247748 A

### SUMMARY OF INVENTION

### TECHNICAL PROBLEM

Although using a ceramic such as silicon nitride, etc., in a biological material has been tried, biocompatibility is still not adequate. Thus, the present disclosure provides a biological material, a powder composition, and a bone prosthetic material which have excellent biocompatibility. Further, the present disclosure provides a method for using a composition containing a composite compound as a biological material having excellent biocompatibility.

### SOLUTION TO PROBLEM

One aspect of the present disclosure provides the biological material described below.
[1] A biological material including, as a main component, a composite compound having yttrium, oxygen, silicon, and nitrogen as constituent elements.
The composite oxide described above has excellent biocompatibility.

The biological material described in [1] may also be any one of [2] to [7] below. The biological materials below have even more excellent biocompatibility.

[2] The biological material described in [1], wherein the composite compound includes at least one compound selected from the group consisting of Si₃N₄-SiO₂-Y₂O₃-based compounds and Si₃N₄-Y₂O₃-based compounds.
The biological material described in [1] or [2], wherein the composite compound has, in a phase diagram of a three-component system of Si₃N₄, SiO₂, and Y₂O₃, a constitution within a triangular region having Y₅Si₃O₁₂N, Y₂Si₃O₃N₄, and Y₄Si₂O₇N₂ as three vertices.
The biological material described in any one of [1] to [3], wherein the composite compound includes at least one compound selected from the group consisting of Y₅Si₃O₁₂N, YSiO₂N, Y₄Si₂O₇N₂, and Y₂Si₃O₃N₄.
The biological material described in any one of [1] to [4], wherein the biological material is powdery and has a BET specific surface area of 0.3 m²/g or more.
The biological material described in any one of [1] to [5], wherein when dispersed in water so as to have a concentration of 6.0 w/v%, a content of ammonium ions included in a liquid phase after ten minutes have elapsed is 0.5 µg/mL or more.
The biological material described in [6], wherein the liquid phase has a pH of 10.0 or less.

One aspect of the present disclosure provides the bone prosthetic material described below.
A bone prosthetic material including the biological material described in any one of [1] to [7].

The bone prosthetic material described above includes a biological material that has excellent biocompatibility. Such a bone prosthetic material is preferred for implants and members of artificial joints, etc.

One aspect of the present disclosure provides the method described below.

[9] A method for using, as a biological material, a composition containing, as a main component, a composite compound having yttrium, oxygen, silicon, and nitrogen as constituent elements.

A composite compound having yttrium, oxygen, silicon, and nitrogen as constituent elements has excellent biocompatibility. Accordingly, such a composite compound can be used preferably as a biological material.

The method described in [9] may also be any one of [10] to [15] below. The biological materials used in the methods below have even more excellent biocompatibility.

[10] The method described in [9], wherein the composite compound includes at least one compound selected from the group consisting of Si₃N₄-SiO₂-Y₂O₃-based compounds and Si₃N₄-Y₂O₃-based compounds.
The method described in [9] or [10], wherein the composite compound has, in a phase diagram of a three-component system of Si₃N₄, SiO₂, and Y₂O₃, a constitution within a triangular region having Y₅Si₃O₁₂N, Y₂Si₃O₃N₄, and Y₄Si₂O₇N₂ as three vertices.
The method described in any one of [9] to [11], wherein the composite compound includes at least one compound selected from the group consisting of Y₅Si₃O₁₂N, YSiO₂N, Y₄Si₂O₇N₂, and Y₂Si₃O₃N₄.
The method described in any one of [9] to [12], wherein the biological material is powdery and has a BET specific surface area of 0.3 m²/g or more.
The method described in any one of [9] to [13], wherein when the biological material is dispersed in water so as to have a concentration of 6.0 w/v%, a content of ammonium ions included in a liquid phase after ten minutes have elapsed is 0.5 µg/mL or more.
The method described in [14], wherein the liquid phase has a pH of 10.0 or less.

One aspect of the present disclosure provides the powder composition described below.

[16] A powder composition including, as a main component, a composite compound having yttrium, oxygen, silicon, and nitrogen as constituent elements, the powder composition having a BET specific surface area of 0.3 m²/g or more.

The powder composition compound described above has excellent biocompatibility.

The powder composition described in [16] may also be any one of [17] to [21] below. The powder compositions below have even more excellent biocompatibility.

[17] The powder composition described in [16], wherein the composite compound includes at least one compound selected from the group consisting of Si₃N₄-SiO₂-Y₂O₃-based compounds and Si₃N₄-Y₂O₃-based compounds.
The powder composition described in [16] or [17], wherein the composite compound has, in a phase diagram of a three-component system of Si₃N₄, SiO₂, and Y₂O₃, a constitution within a triangular region having Y₅Si₃O₁₂N, Y₂Si₃O₃N₄, and Y₄Si₂O₇N₂ as three vertices.
The powder composition described in any one of [16] to [18], wherein the composite compound includes at least one compound selected from the group consisting of Y₅Si₃O₁₂N, YSiO₂N, Y₄Si₂O₇N₂, and Y₂Si₃O₃N₄.
The powder composition described in any one of [16] to [19], wherein when dispersed in water so as to have a concentration of 6.0 w/v%, a content of ammonium ions included in a liquid phase after ten minutes have elapsed is 0.5 µg/mL or more.
The powder composition described in [20], wherein the liquid phase has a pH of 10.0 or less.

### EFFECTS OF INVENTION

According to the present disclosure, it is possible to provide a biological material, a powder composition, and a bone prosthetic material which have excellent biocompatibility. Further, the present disclosure can provide a method for using a composition containing a composite compound as a biological material having excellent biocompatibility.

### BRIEF DESCRIPTION OF DRAWINGS

FIG. 1 is a phase diagram of a three-component system of Si₃N₄, SiO₂, and Y₂O₃.
FIG. 2 is a front view of an example of a bone prosthetic material.

### DESCRIPTION OF EMBODIMENTS

Embodiments of the present disclosure are described below. However, the following embodiments are examples disclosed in order to describe the present disclosure and are not intended to limit the present disclosure to the details described below. It should be noted that numerical value ranges shown using a hyphen "-" include the lower limit value and the upper limit value. That is, a numerical value range shown as "A-B" means A or more and B or less. Numerical value ranges in which the upper limit or lower limit thereof has been replaced with a numerical value from the examples are also included in the present disclosure. When a plurality of materials are disclosed as examples, one of the materials may be used alone or a plurality thereof may be used in combination.

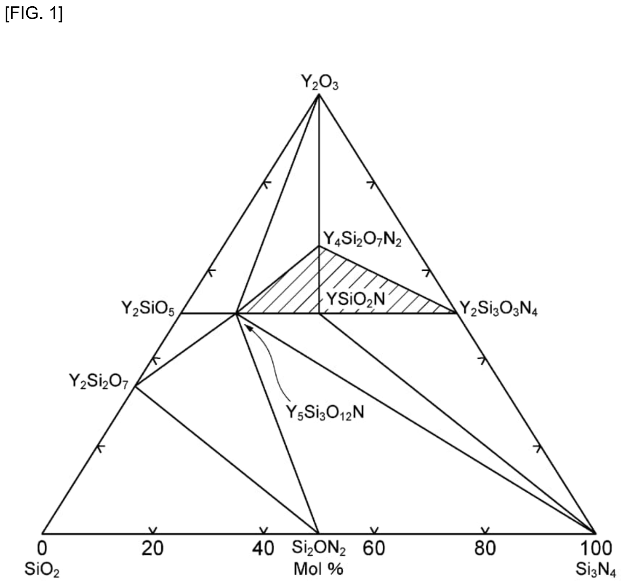

A biological material of one embodiment includes, as a main component, a composite compound having yttrium, oxygen, silicon, and nitrogen as constituent elements. The composite compound may include at least one compound selected from the group consisting of Si₃N₄-SiO₂-Y₂O₃-based compounds and Si₃N₄-Y₂O₃-based compounds. An Si₃N₄-SiO₂-Y₂O₃-based compound is a composite compound which is included in the phase diagram of a three-component system of Si₃N₄, SiO₂, and Y₂O₃ shown in FIG. 1.

The composite compound may have a constitution within the region (including the boundary lines) shown by diagonal shading in FIG. 1. Said region is a triangular region having Y₅Si₃O₁₂N (N-apatite), Y₂Si₃O₃N₄ (N-melilite), and Y₄Si₂O₇N₂ (N-YAM) as three vertices. The triangular region includes constitutions on the boundary lines of the region. The composite compound may include at least one compound selected from the group of Y₅Si₃O₁₂N (N-apatite), YSiO₂N (N-wollastonite), Y₂Si₃O₃N₄ (N-melilite), and Y₄Si₂O₇N₂ (N-YAM), which are on the boundary lines of the triangular region. By doing so, it is possible to sufficiently reduce cell cytotoxicity while also sufficiently raising cell proliferation potential. Further, since osteoconductivity is excellent, it is possible to promote differentiation of undifferentiated mesenchymal cells to osteoblasts. Due thereto, the composite compound is preferably used as a biological material such as a bone prosthetic material, etc.

The biological material may, for example, be granular, may be a powder configured by aggregating a plurality of particles, or may be a solid body such as a film, etc. In the present disclosure, when a plurality of components are included, "main component" refers to a component with the largest content. A component other than the "main component" is referred to as an "accessory component". The biological material may consist solely of a composite oxide as a main component or may include an accessory component which is different from the main component.

The content of the composite oxide in the biological material may be 90 mass% or more, 95 mass% or more, 98 mass% or more, 99 mass% or more, 99.5 mass% or more, or 99.7 mass% or more. The biocompatibility of the biological material tends to improve with a higher content of the composite oxide therein. Examples of an accessory component included in the biological material include silicon oxide, silicon dioxide, yttrium oxide, etc. Further, the biological material may include a composite oxide in which two components among the three foregoing components are composited.

When the biological material is powdery (powder composition), with a small primary particle and a large specific surface area, there is a tendency for both biocompatibility and antibacterial performance to improve. One factor for the improvement in biocompatibility is thought to be because with a large specific surface area, the amount of ammonium ions generated increases when brought into contact with water. One factor for the improvement in antibacterial performance is thought to be because with a large specific surface area, the amount of ammonia generated increases when brought into contact with water. When the specific surface area increases, there is a tendency for antibacterial performance to be improved, in particular with respect to Gram-positive bacteria.

The BET specific surface area of the biological material (powder composition) may be 0.3 m²/g or more, 0.4 m²/g or more, 0.5 m²/g or more, 2.0 m²/g or more, or 4.0 m²/g or more. By increasing the BET specific surface area, it is possible to improve osteoconductivity. If such a biological material is used in an implant or in a member of an artificial joint, etc., differentiation of undifferentiated mesenchymal cells to osteoblasts is promoted and bones can be formed smoothly. The BET specific surface area can be adjusted by changing firing conditions (for example, firing temperature and firing time) when synthesizing the composite oxide, and the size of the raw material powder. The BET specific surface area of the biological material may be 20 m²/g or less, 10 m²/g or less, or 6.0 m²/g or less. Setting such a value makes it possible to improve the handleability of the biological material. As an example, the range of the BET specific surface area is 0.3-20 m²/g. The BET specific surface area can be measured by a method described in the examples.

When the biological material is a powder (in the case of being a powder composition), the average particle diameter (D50, median diameter) may be 25 µm or less, 17 µm or less, 5 µm or less, or 2 µm or less. A small average particle diameter tends to improve biocompatibility and antibacterial performance. This is thought to be because with a small average particle diameter, the amount of ammonium ions and ammonia generated increases due to hydrolysis when brought into contact with water. From the perspective of ease of production and the perspective of improved handleability, the average particle diameter may be 0.2 µm or more, 0.3 µm or more, or 0.5 µm or more. As an example, the range of the average particle diameter is 0.2-25 µm. A particle diameter distribution is measured in compliance with the method described in "particle diameter analysis - laser diffraction/scattering method" of JIS Z 8825: 2013. In a particle diameter distribution (cumulative distribution) with a logarithmic scale particle diameter [µm] shown on the horizontal axis and frequency [vol.%] shown on the vertical axis, the particle diameter at which an integrated value from a small particle diameter reaches 50% of the total is the average particle diameter (D50) described above.

In the particle diameter distribution described above, a particle diameter (D10) at which an integrated value from a small particle diameter reaches 10% of the total may be 0.1-6 µm or 0.2-1 µm. In the particle diameter distribution described above, a particle diameter (D90) at which an integrated value from a small particle diameter reaches 90% of the total may be 1.0-60 µm or 1.0-3.0 µm. D10, D50, and D90 can be adjusted by changing firing conditions (for example, firing temperature and firing time) when performing firing to synthesize the composite oxide, or by changing grinding conditions after firing.

When the biological material is dispersed in water so as to have a concentration of 6.0 w/v%, the pH of the liquid phase (water phase) after ten minutes have elapsed may be 10.0 or less or 9.0 or less. By setting such a value, the concentration of ammonium ions (NH₄⁺) in the liquid phase increases and biocompatibility can be increased even further. The pH of the liquid phase may be 7.5 or more or 8.0 or more. Setting such a value makes it possible to maintain the concentration of ammonia and raise antibacterial performance. The pH of the liquid phase can be adjusted by changing the constitution of the surface of the biological material. For example, if the biological material is treated with hydrofluoric acid, etc., the pH tends to decrease, and thereafter, if washed with water, etc., the pH tends to increase. As an example, the pH of the liquid phase may be 7.5-10.0. When the biological material is dispersed in water so as to have a concentration of 3.6 w/v%, the pH of the liquid phase (water phase) after ten minutes have elapsed may also be the ranges described above.

When the biological material is dispersed in water so as to have a concentration of 6.0 w/v%, the content of ammonium ions included in the liquid phase after ten minutes have elapsed may be 0.5 µg/mL or more, 1.0 µg/mL or more, or 1.5 µg/mL or more. If the content of ammonium ions in the liquid phase is raised, biocompatibility can be improved. The content of ammonium ions may be 4.0 µg/mL or less or 3.0 µg/mL or less. As an example, the range of the content of ammonium ions included in the liquid phase may be 0.5-4.0 µg/mL. When the biological material is dispersed in water so as to have a concentration of 3.6 w/v%, the content of ammonium ions included in the liquid phase after ten minutes have elapsed may also be the ranges described above.

When the biological material is dispersed in water so as to have a concentration of 6.0 w/v%, the content of ammonia included in the liquid phase after ten minutes have elapsed may be 0.1 µg/mL or more or 0.2 µg/mL or more. Such a biological material can also have antibacterial properties. The content of ammonia may be 1.0 µg/mL or less or 0.8 µg/mL or less. As an example, the range of content of ammonia included in the liquid phase may be 0.1-1.0 µg/mL. Herein, the unit "w/v%" is synonymous with "g/100 mL". The contents of ammonia and ammonium ions in the liquid phase and the pH are measured by a method described in the examples under conditions of atmospheric pressure and a temperature of 20°C. When the biological material is dispersed in water so as to have a concentration of 3.6 w/v%, the content of ammonia included in the liquid phase after ten minutes have elapsed may also be the ranges described above.

The biological material can, for example, be produced by the following procedure. A silicon nitride powder, an yttria powder, and a silica powder are prepared as raw material powders. A mixed powder is obtained by blending at least two of the foregoing powders so as to obtain a target crystal composition. The raw material powders may be mixed by using, for example, a ball mill, etc. The raw material powders may be wet-blended by using a solvent. The mixed powder is sieved and, after removing coarse grain, is fired in a nitrogen atmosphere at a temperature of 1500-1600°C. The firing time may, for example, be 2-20 hours. By subjecting the obtained fired product to a crushing process using a ball mill, etc., it is possible to obtain a powdery biological material including, as a main component, a composite compound having yttrium, oxygen, silicon, and nitrogen as constituent elements. The crystalline phase included in the biological material can be analyzed by X-ray diffraction.

The biological material of the present embodiment has excellent antibacterial properties with respect to Gram-positive bacteria. The form of the biological material is not particularly limited and may, for example, be a powder, bulk, thin film, or coating. The biological material may, for example, be coated on a biomedical implant.

A powder composition according to one embodiment includes, as a main component, a composite compound having yttrium, oxygen, silicon, and nitrogen as constituent elements, and the powder composition has a BET specific surface area of 0.3 m²/g or more. The powder composition may have the same constitution, size, and characteristics as the powdery biological material. The details described in the embodiment of a biological material also apply to the powder composition of the present embodiment. The powder composition has excellent biocompatibility, and therefore, can be preferably used as a biological material.

A use method according to one embodiment uses, as a biological material, a composition containing, as a main component, a composite compound having yttrium, oxygen, silicon, and nitrogen as constituent elements. Composite oxides included as a main component in the composition are as described in the embodiment of a biological material. The contents of the main component and accessory components in the composition are as described in the embodiment of a biological material. The details described in the embodiment of a composite oxide also apply to the use method of the present embodiment. That is, the composition may contain the same components as the biological material described above and may have the same form and characteristics as the biological material described above. The form of the composition is not particularly limited and may, for example, be a powder, bulk, thin film, or coating. The composition may be dispersed in a matrix such as a film or filter, etc. The composition used in the use method of the present embodiment has excellent biocompatibility, and therefore, can be preferably used as a biological material.

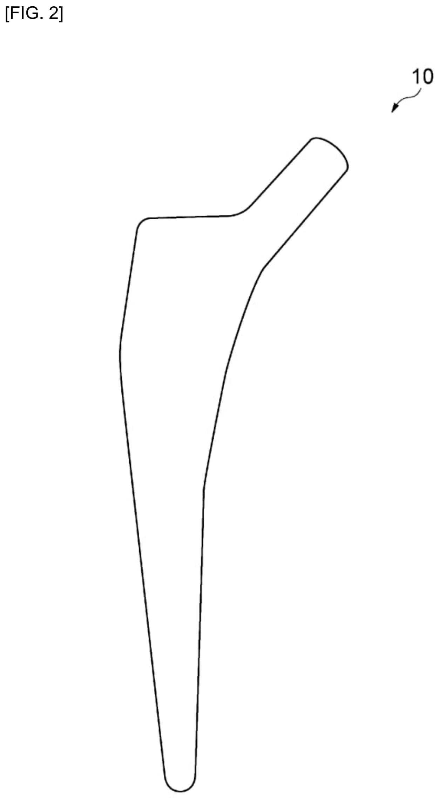

A bone prosthetic material according to one embodiment includes the biological material described above. The bone prosthetic material may, for example, be an implant, a component 10 (stem) of an artificial joint such as that shown in FIG. 2, or a member that is inserted into a part having a bone defect. The bone prosthetic material may be a paste material. The biological material may be coated on at least a portion of a surface of a substrate of the bone prosthetic material or may be dispersed in the bone prosthetic material.

Embodiments of the present disclosure were described above, but the present disclosure is not in any way limited to the above embodiments.

### EXAMPLES

The features of the present disclosure are explained in more detail with reference to examples and comparative examples. However, the present disclosure is not limited to the examples below.

### (Example 1)

[Preparation of biological material] A biological material was prepared by the procedure described below. As raw material powders, 0.7 g of a silicon nitride powder (product name: E10, manufactured by UBE Corporation), 11.5 g of an yttria powder (product name: UUHP, manufactured by Shin-Etsu Chemical Co., Ltd.), and 2.8 g of a silica powder (manufactured by Kojundo Chemical Laboratory Co., Ltd.) were weighed. The weighed raw material powders, 350 g of balls made of silicon nitride and having a diameter of 16 mm, and 150 ml of ethanol were put into a container (Nalgene plastic bottle, capacity 500 ml). The lid of the container was sealed and the container was placed in a tabletop ball mill (product name: BM-15, manufactured by Eishin Co., Ltd.). The rotation speed was set to 82 rpm and pre-mixing was performed for one hour under these conditions to obtain a mixed slurry.

A membrane filter was used to capture only the powder from the obtained mixed slurry. The captured particles were dried for three hours at 120°C. After being dried, the powder was sieved using a nylon sieve (#100). The minus sieve powder was packed in a molding container made of boron nitride. The molding container was fired for six hours under conditions of 1550°C, a nitrogen atmosphere, and 0.85 MPa (gauge pressure) using an electric furnace (product name: Hi Multi 500, manufactured by Fujidempa Kogyo Co., Ltd.).

The powder obtained by the firing was put into a container (Nalgene plastic bottle, capacity 500 ml). 375 g of balls made of silicon nitride and having a diameter of 5 mm and 150 ml of ethanol were put into the container and then the lid of the container was sealed. The sealed container was placed in a tabletop ball mill, the rotation speed was set to 82 rpm, and a crushing process was performed for six hours under these conditions. A membrane filter was used to capture powder from the slurry after the crushing process. The captured particles were dried for three hours at 110°C. After being dried, the powder was sieved using a nylon sieve (#330). The minus sieve powder was used to obtain the Si₃N₄-SiO₂-Y₂O₃-based compound of Example 1. An X-ray diffraction device (device name: Ultima IV, manufactured by Rigaku Corporation) was used to investigate the components of the powder. As a result thereof, it was confirmed that the powder includes N-apatite (Y₅Si₃O₁₂N) as a main component. Meanwhile, components other than N-apatite were not detected. The powder was used as the biological material (powder composition) of Example 1.

### [Evaluation of biological material]

### <Particle diameter distribution>

The particle diameter distribution of the biological material was measured by a laser-diffraction/scattering method. Measurement was performed in compliance with the method described in JIS Z 8825: 2013 "Particle diameter analysis - laser diffraction/scattering method". In a particle diameter distribution (cumulative distribution) with a logarithmic scale particle diameter [µm] shown on the horizontal axis and frequency [vol.%] shown on the vertical axis, the particle diameters at which an integrated value from a small particle diameter reaches 10%, 50%, and 90% of the total were respectively determined as D10, D50, and D90. The results are shown in Table 1.

### <BET specific surface area>

The BET specific surface area of the biological material was measured by a single point BET method using nitrogen gas in compliance with JIS Z 8830: 2013 "Method for measuring specific surface area of powder (solid) by gas adsorption". The results are shown in the "SSA" row in Table 1.

### <Evaluation of liquid phase>

30 mL of distilled water was put into a vial having a capacity of 50 mL. 1.8 g of the biological material was put into the vial and stirred for 30 seconds to obtain a dispersion liquid. Thereafter, the lid of the vial was closed and the dispersion liquid was left for ten minutes. After ten minutes had elapsed, filter paper having a mesh size of 5.0 µm was used to filter the dispersion liquid to obtain a filtrate (liquid phase). The foregoing actions were performed at atmospheric pressure in a 20°C environment.

The respective quantities of ammonia (NH₃) and ammonium ions (NH₄⁺) in the filtrate were determined by an ion chromatograph (device name: ICS-2100, manufactured by Thermo Fisher Scientific Inc.). A pH meter (device name: FP20-Std-Kit, manufactured by Mettler Toledo) was used to measure the pH of the filtrate. The contents of ammonia (NH₃) and ammonium ions (NH₄⁺) in the filtrate, and the pH are shown in the "LIQUID PHASE" row of Table 1.

### <Antibacterial properties>

A bacterial suspension (1×10⁵ to 1×10⁶ CFU/mL) was prepared by pre-culturing bacteria (*Staphylococcus aureus*) in a BHI liquid culture medium. A 15 w/v% dispersion liquid was obtained by putting 0.15 g of the biological material and 1 mL of distilled water into a microtube. After sterilizing the microtube with ultraviolet light, 1 mL of the bacterial suspension described above was added to the microtube. Then, after mixing for five minutes at room temperature (approximately 20°C) in a tube rotator, the supernatant was collected and the viability of the bacteria was measured by using a WST-8 assay and fluorescent imaging (Live & Dead staining: CFDA·PI·DAPI). Viability was also measured in the same manner when *Staphylococcus epidermidis* was used as the bacteria. The results are shown in Table 1. Viability results when the biological material was not used are shown in the "REF." column of Table 1.

### <Biocompatibility>

After being ground with a mortar and pestle, the biological material was sieved so as to achieve a material with a Φ of 75 µm or less. Ultrapure water was used to prepare suspensions in which the concentrations of biocompatible material therein were, respectively, 1 mg/mL and 10 mg/mL. The suspensions were dripped onto tissue culturing microplates [Iwaki 3820-024 (24 wells) or MatTek P06G-0-20-F/H (6 wells)] so as to be, respectively, 0.1 mg/well and 1 mg/well. After the dripping, the microplates were dried for 24 hours at 50°C in a dry heat sterilizer (TABAI LC-122) and sterilized with ultraviolet light. Thus, cell culturing beds were fabricated. A growth medium and an osteoblast differentiation medium were prepared on the beds that were fabricated in the manner described above. Methods for preparing the media were as described below.

A medium having DMEM with 4.5 g/L glucose (Nacalai tesque), 10% of fetal bovine serum, and 1% of PS added thereto was used as the growth medium. Further, a medium obtained by adding 10 mM of β-glycerophosphate, 50 µg/mL of (L+) ascorbic acid, and 100 nM of dexamethasone to the growth medium prepared as described above was used as the osteoblast differentiation medium.

A mouse bone marrow-derived KUSA-A1 cell line (JRCB) was made a first choice. The KUSA-A1 cell line was seeded, at a cell number of 5×10⁴/cm², in each of the growth medium and the osteoblast differentiation medium, and cell proliferation potential and cell viability (cytotoxicity) were evaluated after three days, after seven days, and after 14 days.

WST-8 (product name: Dojindo 341-07761, manufactured by Fujifilm Wako Pure Chemical Corporation) was used in the evaluation of cell proliferation potential. The method for measuring WST-8 was performed in accordance with the instructions accompanying the product. Sample absorbance after 14 days was measured (product name: Spectrophotometer U-3900, manufactured by Hitachi High-Tech Corporation) to quantify the cell proliferation potential. The results are shown in Table 1. A larger absorbance indicates a superior cell proliferation potential.

Live or dead staining (product name: Catsha-pu L3224, Thermofisher Scientific) was used in the evaluation of cell viability (cytotoxicity). The live or dead staining measuring method was performed in accordance with the instructions accompanying the product. Surfaces of the media after 14 days were observed using an optical microscope and evaluated on the basis of the following criteria. Comparative Example 1 shall be described later. The results are shown in Table 1.
A: Smaller ratio of yellow and red sections than in Comparative Example 1.
B: Ratio of yellow and red sections equal to that in Comparative Example 1.
C: Larger ratio of yellow and red sections than in Comparative Example 1.

Osteoblast differentiation induction (calcification) performance was estimated using Alizarin Red S staining to make an evaluation. The Raman spectrum of the media after staining was measured and the degree of staining was evaluated on the basis of the following criteria.
A: Larger ratio of stained sections than in Comparative Example 1.
B: Ratio of stained sections equal to that in Comparative Example 1.
C: Smaller ratio of stained sections than in Comparative Example 1.

### (Example 2)

5 g of the biological material (powder including N-apatite as a main component) of Example 1 was put into a container (Nalgene plastic bottle, capacity 500 ml), and then 375 g of silicon nitride balls with a diameter of 5 mm and 150 ml of ethanol were put in, the lid of the container was closed, and a crushing process (rotation speed: 82 rpm, processing time: 24 hours) was performed with the tabletop ball mill used in Example 1. The powder after the crushing process was used as the biological material of Example 2. Evaluations of the biological material were performed in the same manner as for Example 1. The results are shown in Table 1.

### (Example 3)

A biological material was prepared by the procedure described below. 3.2 g of a silicon nitride powder (product name: E10, manufactured by UBE Corporation), 10.4 g of an yttria powder (product name: UUHP, manufactured by Shin-Etsu Chemical Co., Ltd.), and 1.4 g of a silica powder (manufactured by Kojundo Chemical Laboratory Co., Ltd.) were weighed. Other than using the foregoing raw material powders, the Si₃N₄-SiO₂-Y₂O₃-based compound powder of Example 3 was obtained by performing the same procedure as in Example 1. The components of the powder were investigated in the same manner as in Example 1. As a result thereof, it was confirmed that the powder includes N-wollastonite (YsiO₂N) as a main component. Meanwhile, components other than N-wollastonite were not detected. The powder was used as the biological material of Example 3. An evaluation of the biological material of Example 3 was performed by the same procedure as in Example 1. The evaluation results are shown in Table 1.

### (Example 4)

5 g of the biological material (powder including N-wollastonite as a main component) of Example 3 was put into a container (Nalgene plastic bottle, capacity 500 ml), and then 375 g of silicon nitride balls with a diameter of 5 mm and 150 ml of ethanol were put in, the lid of the container was closed, and a crushing process (rotation speed: 82 rpm, processing time: 24 hours) was performed with the tabletop ball mill used in Example 1. The powder after the crushing process was used as the biological material of Example 4. Evaluations of the biological material were performed in the same manner as for Example 1, The results are shown in Table 1.

### (Example 5)

A biological material was prepared by the procedure described below. 1.9 g of a silicon nitride powder (product name: E10, manufactured by UBE Corporation), 12.3 g of an yttria powder (product name: UUHP, manufactured by Shin-Etsu Chemical Co., Ltd.), and 0.8 g of a silica powder (manufactured by Kojundo Chemical Laboratory Co., Ltd.) were weighed. Other than using the foregoing raw material powders, the Si₃N₄-SiO₂-Y₂O₃-based compound powder of Example 5 was obtained by performing the same procedure as in Example 1. The components of the powder were investigated in the same manner as in Example 1. As a result thereof, it was confirmed that the powder includes N-YAM (Y₄Si₂O₇N₂) as a main component. Meanwhile, components other than N-YAM were not detected. The powder was used as the biological material of Example 5. An evaluation of the biological material of Example 5 was carried out using the same procedure as in Example 1. The evaluation results are shown in Table 2.

### (Example 6)

A biological material was prepared by the procedure described below. 5.7 g of a silicon nitride powder (product name: E10, manufactured by UBE Corporation) and 9.3 g of an yttria powder (product name: UUHP, manufactured by Shin-Etsu Chemical Co., Ltd.) were weighed. Other than using the foregoing raw material powders, the Si₃N₄-Y₂O₃-based compound powder of Example 6 was obtained by performing the same procedure as in Example 1. The components of the powder were investigated in the same manner as in Example 1. As a result thereof, it was confirmed that the powder includes N-melilite (Y₂Si₃O₃N₄) as a main component. Meanwhile, components other than N-melilite were not detected. The powder was used as the biological material of Example 6. An evaluation of the biological material of Example 6 was carried out using the same procedure as in Example 1. The evaluation results are shown in Table 2.

### (Comparative Example 1)

In the evaluation of biocompatibility, other than not using the biological material, cell proliferation potential, cytotoxicity, and calcification were evaluated using the same procedures as in Example 1. The evaluation results were as shown in Table 1. Note that in evaluating cytotoxicity and calcification, other examples and a comparative example were evaluated with the results of Comparative Example 1 serving as a reference therefor.

### (Comparative Example 2)

A commercially sold silicon nitride powder (product name: E10, manufactured by UBE Corporation) was used as the biological material of Comparative Example 2. An evaluation of the biological material of Comparative Example 2 was performed by using the same procedure as in Example 1. The evaluation results are shown in Table 2.

**TABLE 1**

| SAMPLE | ITEM | | REF. | EX.1 | EX.2 | EX.3 | EX.4 |
|---|---|---|---|---|---|---|---|
| BIOLOGICAL MATERIAL | D10 | [µm] | - | 3.53 | 0.70 | 3.53 | 0.60 |
| | D50 | [µm] | - | 16.36 | 1.20 | 11.70 | 1.20 |
| | D90 | [µm] | - | 52.40 | 1.80 | 39.74 | 1.90 |
| | SSA | [m²/g] | - | 0.44 | 4.36 | 0.51 | 4.26 |
| LIQUID PHASE | NH₃ | [µg/mL] | - | 0.15 | 0.29 | 0.50 | 0.28 |
| | NH₄⁺ | [µg/mL] | - | 0.31 | 2.50 | 0.96 | 1.74 |
| | pH | | - | 9.65 | 8.49 | 9.31 | 8.69 |
| ANTIBACTERIAL PROPERTIES | STAPHYLOCOCCUS AUREUS | | 0.72 | 0.37 | 0.38 | 0.36 | 0.02 |
| | STAPHYLOCOCCUS EPIDERMIDIS | | 2.00 | 1.40 | 0.10 | 1.60 | 0.05 |
| BIOCOMPATIBILITY | CELL PROLIFERATION POTENTIAL | | - | 1.52 | 1.42 | 1.82 | 1.40 |
| | CYTOTOXICITY | | - | A | A | A | A |
| | CALCIFICATION | | - | B | A | B | A |

**TABLE 2**

| SAMPLE | ITEM | | EX.5 | EX.6 | COMP. EX.1 | COMP. EX.2 |
|---|---|---|---|---|---|---|
| BIOLOGICAL MATERIAL | D10 | [µm] | 5.54 | 3.36 | - | 0.16 |
| | D50 | [µm] | 23.83 | 17.74 | - | 0.84 |
| | D90 | [µm] | 55.06 | 53.52 | - | 1.53 |
| | SSA | [m²/g] | 0.34 | 0.49 | - | 11.11 |
| LIQUID PHASE | NH₃ | [µg/mL] | 0.23 | 0.30 | - | 0.65 |
| | NH₄⁺ | [µg/mL] | 0.31 | 0.52 | - | 2.97 |
| | pH | | 9.27 | 9.42 | - | 8.858 |
| ANTIBACTERIAL PROPERTIES | STAPHYLOCOCCUS AUREUS | | 0.74 | 0.75 | - | 0.05 |
| | STAPHYLOCOCCUS EPIDERMIDIS | | 1.77 | 1.86 | - | 0.05 |
| BIOCOMPATIBILITY | CELL PROLIFERATION POTENTIAL | 1.52 | 1.48 | 0.81 | 1.11 | |
| | CYTOTOXICITY | A | A | REF | B | |
| | CALCIFICATION | B | B | REF | C | |

In Tables 1 and 2, "-" means not measured. As shown in Table 1 and Table 2, it was confirmed that the biological materials of Examples 1-6 all have excellent cell proliferation potential and also that cytotoxicity is sufficiently suppressed. From comparisons with Examples 1 and 2 and comparisons with Examples 3 and 4, it was confirmed that by increasing the specific surface area, differentiation to osteoblasts is promoted and antibacterial properties with respect to Gram-positive bacteria (in particular, *Staphylococcus epidermidis*) improve.

### INDUSTRIAL APPLICABILITY

According to the present disclosure, it is possible to provide a biological material, a powder composition, and a bone prosthetic material which have excellent biocompatibility. It is possible to provide a method for using a composition containing a composite compound as a biological material having excellent biocompatibility.

### REFERENCE SIGNS LIST

- 10: Component (stem)

## Claims

1. A biological material comprising, as a main component, a composite compound having yttrium, oxygen, silicon, and nitrogen as constituent elements.

2. The biological material according to claim 1, wherein the composite compound comprises at least one compound selected from the group consisting of Si₃N₄-SiO₂-Y₂O₃-based compounds and Si₃N₄-Y₂O₃-based compounds.

3. The biological material according to claim 1, wherein the composite compound has, in a phase diagram of a three-component system of Si₃N₄, SiO₂, and Y₂O₃, a constitution within a triangular region having Y₅Si₃O₁₂N, Y₂Si₃O₃N₄, and Y₄Si₂O₇N₂ as three vertices.

4. The biological material according to claim 1, wherein the composite compound comprises at least one compound selected from the group consisting of Y₅Si₃O₁₂N, YSiO₂N, Y₄Si₂O₇N₂, and Y₂Si₃O₃N₄.

5. The biological material according to claim 1, wherein the biological material is powdery and has a BET specific surface area of 0.3 m²/g or more.

6. The biological material according to claim 1, wherein when dispersed in water so as to have a concentration of 6.0 w/v%, a content of ammonium ions included in a liquid phase after ten minutes have elapsed is 0.5 µg/mL or more.

7. The biological material according to claim 6, wherein the liquid phase has a pH of 10.0 or less.

8. A bone prosthetic material comprising the biological material according to any one of claims 1 to 7.

9. A method for using, as a biological material, a composition containing, as a main component, a composite compound having yttrium, oxygen, silicon, and nitrogen as constituent elements.

10. A powder composition comprising, as a main component, a composite compound having yttrium, oxygen, silicon, and nitrogen as constituent elements, the powder composition having a BET specific surface area of 0.3 m²/g or more.
